# EUROPÄISCHE PATENTSCHRIFT

(11) **EP 1 754 973 B1**
(45) Veröffentlichungstag und Bekanntmachung des Hinweises auf die Patenterteilung: **03.11.2010**
(21) Anmeldenummer: 06015279.0
(22) Anmeldetag: 21.07.2006
(51) Int. Cl.: G01N 35/02, G01N 33/04, G01N 1/18

(54) **Verfahren und Vorrichtung zur Probennahme und unmittelbaren Analyse fliessfähiger Sammelgutmengen, insbesondere von Milchlieferungen**
Method and device for sampling and directly analysing collected flowable material, in particular milk deliveries
Méthode et dispositif pour l'échantillonnage et l'analyse immédiate d'un fluide collecté, en particulier lors de livraison de lait

(30) Priorität: 26.07.2005 DE 102005035506
(43) Veröffentlichungstag der Anmeldung: 21.02.2007
(73) Patentinhaber: Raudszus Electronic GmbH, 94239 Ruhmannsfelden (DE)
(72) Erfinder: Raudszus, Gerhard, 94239 Zachenberg/ Giggenried (DE)
(74) Vertreter: Benninger, Johannes

(56) Entgegenhaltungen:
- EP-A- 0 502 638
- EP-A- 1 475 152
- EP-B- 0 186 944
- EP-B- 0 490 447
- WO-A-02/18956
- WO-A-95/08774
- WO-A-2004/102183
- DE-A1- 3 402 304
- DE-A1- 19 930 956
- GB-A- 2 250 590
- US-A- 4 292 994
- US-A1- 2003 215 364
- US-B1- 6 297 045

## Beschreibung

Die vorliegende Erfindung betrifft ein Verfahren zur Probennahme und unmittelbaren Analyse fließfähiger Sammelgutmengen, insbesondere von Milchlieferungen.

### Stand der Technik

Bestimmte dezentral produzierte Güter, bspw. landwirtschaftlich produzierte Güter, werden vom Lieferanten, bspw. einem landwirtschaftlichen Betrieb, in bestimmten Gutmengen in Anlieferungsbehälter abgefüllt und an Lieferstellen zur Verfügung gestellt, um dort durch Sammeltransportfahrzeuge eingesammelt zu werden. Die in einem Sammeltransportfahrzeug vereinten Gutmengen werden zu einer Sammelstelle, bspw. einer Molkerei, transportiert und dort mit den Gutmengen anderer Sammeltransportfahrzeuge vereinigt.

Eine Qualitätsüberprüfung der einzelnen Lieferungen ist hierbei unabdingbar. Zur Qualitätsbeurteilung von Lieferungen wird in der Regel vor, während oder nach der Überführung des Liefergutes aus dem Anlieferungsbehälter in den Sammelbehälter eine repräsentative Probe des Liefergutes entnommen. Eine eingehende Analyse dieser Probe kann erst nach dem Transport in ein Labor durch fachmännisches Personal erfolgen.

Es ist jedoch von höchster wirtschaftlicher Bedeutung, die Qualität der einzelnen Lieferungen so schnell wie möglich, am besten unmittelbar im Anschluss und am Ort der Probennahme, beurteilen zu können, um eine eventuelle Verunreinigung der gesamten Sammelmenge durch einen Lieferanteil von schlechter Qualität zu verhindern und damit die Qualität des Gesamtproduktes sicherzustellen. Deshalb beschäftigt sich die vorliegende Erfindung mit einem einfachen Testsystem, das eine mobile, von einem Labor unabhängige und von einem Nicht-Fachmann durchführbare Erstanalyse am Ort der Probennahme möglich macht. Eine derartige Erstanalyse ist insbesondere bei sensiblen Produkten notwendig, die einerseits einem schnellen natürlichen Verfall, andererseits vielfältigen Möglichkeiten einer Qualltätsbeeinträchtigung unterliegen, Die vorliegende Erfindung richtet sich daher insbesondere auf die Milchwirtschaft. Diese Anwendungsmöglichkeit ist jedoch nicht ausschließlich zu verstehen; eine Anwendung bei der Sammlung anderer fließfähiger Produkte, bspw. Rapsöl o. ä., ist grundsätzlich ebenfalls möglich. Dies ist auch dann der Fall, wenn sich die vorliegende Beschreibung vorwlegend auf Milchlieferungen bezieht.

Bislang wird eine erste Analyse von Sammelgutlieferungen, insbesondere von Milchlieferungen, manuell vom Führer des Sammeltransportfahrzeuges durchgeführt. Im Falle von Milchlieferungen handelt es sich bei dieser Erstanalyse insbesondere um einen Test auf das Vorhandensein bestimmter Antibiotika (bspw. β-Lactam-Antibiotika) im Liefervolumen. Der Durchführende des Tests entnimmt vor, während oder nach dem Einsammeln der Liefermenge eine Analyseprobe und führt den Test durch. Dieses Vorgehen ist einerseits zeitaufwändig. Andererseits birgt es die üblichen Gefahren des menschlichen Vorgehens: die Analyse kann vergessen werden, sie kann falsch (bspw. unsteril) durchgeführt oder falsch zugeordnet werden, etc.; nicht zuletzt kann sie manipuliert werden.

Aus US-A-4 292 994 ist das Befüllen eines markierten leeren Behälters mit einer zu analysierenden Probe von Milch während des Überführens von dem Lieferbehälter in den Sammeltransportbehälter und darauffolgende Analyse in dem milchwirtschaftlichen Betrieb bekannt.

Aus EP-B-0 186 944 ist ein Verfahren und eine Ausrüstung für Antibiotikaprüfung in einer flüssigen Probe wie Milch, in der die Milch mit einem trockenen Reagens und einem Reagenssystem, das eine Farbreaktion produziert, gemischt wird bekannt. Das trockene Reagens wird auf einem Träger immobilisiert. Das Verfahren ist besonders geeignet für den Bauer oder den Fahrer eines Sammeltransportfahrzeuges.

### Aufgabenstellung

Ein vorrangiges Ziel der Erfindung besteht darin, ein Verfahren und eine Vorrichtung zur Verfügung zu stellen, die ein Sammeln von Analyseproben und eine unmittelbar an die Probennahme anschließende Erstanalyse dieser Proben ermöglichen. Das Verfahren und die Vorrichtung sollen hierbei im Sammeltransportfahrzeug an- bzw. verwendbar sein. Sowohl die Probennahme als auch die unmittelbar nachfolgende Analyse sollen vorzugsweise durch Personen ohne besondere Fähigkeiten durchführbar sein, insbesondere automatisiert ablaufen. Die Ergebnisdaten sollen vorzugsweise unmittelbar zur Verfügung stehen. Eine eindeutige Zuordnung der Proben und Ergebnisdaten zu den Liefergutfraktionen und damit zu den Lieferanten soll gewährleislet sein. Ein menschliches Eingreifen In die Probennahme und den Test soll nicht erforderlich und gegebenenfalls unterbindbar sein.

Diese Ziele der Erfindung werden mit, dem Gegenstand des Anspruchs 1 erreicht. Merkmale vorteilhafter Weiterbildungen der Erfindung lassen sich den abhängigen Ansprüchen entnehmen.

In der folgenden Beschreibung werden die erfindungsgemäßen Verfahrensschritte im Zusammenhang dargestellt und im Detail erklärt.

Sämtliche im Zuge der vorliegenden Erfindung verwendeten Behälter sind vorzugsweise während des gesamten Verfahrens jeweils mit einer Abdeckung versehen, die die Verunreinigung der Behälterinhalte verhindert und ein Befüllen bzw. eine Entnahme nur in geregelter Weise zulässt. Eine derartige Abdeckung weist bevorzugt eine durchstechbare Fläche auf, die bspw. aus Silikon bestehen kann. Eine derartige Abdeckung, insbesondere eine Silikonabdeckung weist den Vorteil auf, dass sie leicht bspw. mit einer Kanüle durchstochen werden kann, und nach Herausziehen der Kanüle wieder einen luftdichten Verschluss des Behälters darstellt. Andere Materialien mit ähnlichen Eigenschaften können für diesen Zweck ebenfalls verwendet werden.

Das Befüllen des wenigstens einen bereitgestellten, zunächst leeren Behälters mit einer zu analysierenden Probe des jeweiligen Lieferguts sowie die Entnahme vorgebbarer Mengen aus diesen Proben sowie die Entnahme der Reaktions- oder auch Relnigungsflüssigkeit aus den jeweiligen Behältern erfolgt vorzugsweise durch eine Kanüle, mit der die jeweilige Abdeckung durchstochen wird. Die Kanüle steht hierzu mit einer Überführungseinrichtung in Verbindung. Diese kann bspw. eine Pumpe, eine Einweg- oder Mehrwegspritze, eine Pipette o. ä. sein.

Das erfindungsgemäße Verfahren sieht vor, dass eine vorgebbare Menge des Lieferguts während der Überführung des Lieferguts vom Lieferbehälter in den Sammelgutbehälter in den wenigstens einen bereitgestellten, sauberen, insbesondere sterilen, leeren Behälter überführt wird. Dies geschieht durch Abzweigen der Probe aus der Einrichtung, welche der Überführung des Lieferguts vom Lieferbehälter in den Sammelgutbehälter dient (bspw. dem Pumpschlauch). Bspw. kann hieran über ein T-Stück mit Ventil ein weiterer Schlauch angeschlossen sein, durch den die Probe abgezweigt wird. Dies geschieht insbesondere durch Anschluss einer Pumpe, bspw. einer Peristaltikpumpe, die den Vorteil aufweist, auf besonders einfache Weise sauber bzw. steril zu arbeiten. Die Verwendung anderer Pumpvorrichtungen ist ebenso möglich. Die derart gesammelte, vorgebbare Menge dient als repräsentative Probe des Lieferguts, die nachfolgend verschiedenen Analysen zur Qualitätsbestimmung unterzogen wird. Wenigstens eine dieser Analysen kann erfindungsgemäß unmittelbar im Anschluss an die Probennahme und am Ort der Probennahme, insbesondere im Sammeltransportfahrzeug, durchgeführt werden. Die verbleibende Menge Probe kann, je nach Art des Gutes, gekühlt oder nicht gekühlt zur Sammelstelle bzw. zu einem Labor transportiert und dort weiteren Analysen unterzogen werden.

Bei Anwendung des erfindungsgemäßen Verfahrens in der Milchindustrie ist die erste, am Ort und zum Zeitpunkt der Liefergutaufnahme durchzuführende Analyse bevorzugt ein Test auf das Vorhandensein von Antibiotika in der Milch. Hierbei wird insbesondere auf das Vorhandensein von β-Lactam-Antibiotika getestet. Das erfindungsgemäße Verfahren ermöglicht jedoch auch andere Analysen unter Verwendung anderer als der genannten Nachweissysteme, die je nach Anforderung gewählt sein können.

Gemäß einer bevorzugten Ausführungsform der Erfindung sieht das erfindungsgemäße Verfahren vor, dass zur Durchführung der Analyse eine Reaktionsflüssigkeit bereitgestellt ist, Das Bereitstellen einer Einrichtung mit einer Reaktionskomponente umfasst in diesem Falle:
- Bereitstellen einer vorgebbaren Menge Reaktionsflüssigkeit;
- Hinzugeben einer vorgebbaren Menge der zu analysierenden Probe;
- Inkubation der Reaktionsmischung entsprechend vorgebbarer Inkubationsbedingungen;

Die Reaktionsflüssigkeit enthält bevorzugt alle Komponenten, die für die gewünschte Analysemethode notwendig sind, außer der zu analysierenden Probe selbst. Für Nachweisverfahren, die insbesondere auf der spezifischen Bindung detektierbarer Moleküle an zu detektierende Substanzen beruhen, beinhaltet die Reaktionsflüssigkeit die bindenden Moleküle, insbesondere Rezeptormoleküle. Im Falle einer Anwendung des Verfahrens in der Milchindustrie, also bspw. zum Nachweis von β-Lactam-Antibiotika in Rohmilch, weist die Reaktionsflüssigkeit neben einer geeigneten Puffersubstanz auch Rezeptormoleküle auf, die insbesondere spezifisch an β-Lactam-Antibiotika binden (bspw. PBP, Penicillin bindende Proteine). Derartige Reaktionsmischungen, Rezeptormoleküle und Nachweissysteme sind im Stand der Technik bekannt. Auch Nachweissysteme für andere Substanzen sind bekannt und können im Rahmen der vorliegenden Erfindung durchgeführt werden.

Gemäß dem erfindungsgemäßen Verfahren wird zu Beginn der Durchführung der Analyse eine vorgebbare Menge der bereitgestellten Menge Reaktionsflüssigkeit dem entsprechenden Behälter entnommen. Diese Entnahme kann wie beschrieben mittels einer Kanüle erfolgen, mit der die Abdeckung des Behälters durchstochen wird. Die hierbei wirkende Überführungseinrichtung, welche mit der Kanüle in Verbindung steht, kann eine Pipette oder Spritze sein, welche die entsprechende Menge Reaktionsflüssigkeit aufnimmt Die vorgebbare Menge an Reaktionsflüssigkeit (bspw. 25 µl) kann aus der Überführungseinrichtung in ein Reaktionsgefäß abgegeben werden, oder In der Überführungseinrichtung verbleiben, so dass diese innerhalb der Überführungseinrichtung bereitgestellt wird. Alternativ kann eine vorgebbare Menge Reaktionsflüssigkeit bereits im Zuge der Analysenvorbereitungen, also bereits bevor das Sammeltransportfahrzeug die Lieferungen einsammelt, in einem Reaktionsgefäß vorgelegt sein.

Im Anschluss daran erfolgt erfindungsgemäß ein Zusammenführen zumindest eines Teilvolumens der zu analysierenden Probe mit der Reaktionskomponente, in diesem Fall also mit der vorgelegten Reaktionsflüssigkeit.

Hierzu wird erfindungsgemäß, wiederum vorzugsweise mittels einer Kanüle und einer damit gekoppelten Überführungseinrichtung, eine vorgebbare Menge (bspw. 150 µl) der zu analysierenden Probe entnommen und diese mit der bereitgesteilten Reaktionsflüssigkeit in einem sich aus den jeweiligen Mengen ergebenden bestimmten Mischungsverhältnis gemischt. Im dem Fall, dass die Reaktionsflüssigkeit in einem Reaktionsgefäß vorgelegt ist, kann die vorgebbare Menge der zu analysierenden Probe einfach hinzugefügt werden. Das Mischen kann bspw. erfolgen, indem die Kanüle, durch die die Probe hinzugefügt wird, in die vorgelegte Flüssigkeit eintaucht, Ein besseres Mischen kann erzielt werden, indem die so hergestellte Reaktionsmischung nochmals in die Überführungseinrichtung eingesaugt und anschließend in das Reaktionsgefäß zurückgegeben wird. Dieses Mischverfahren ist im Stand der Technik bekannt. Im alternativen Fall wird die zu analysierende Probe im Anschluss an die Aufnahme der Reaktionsflüssigkeit in die Überführungseinrichtung in dieselbe aufgenommen. Dies geschieht wiederum vorzugsweise indem die Kanüle die Abdeckung des entsprechenden Behälters durchsticht und eine vorgebbare Menge der Probe durch Betätigen der Überführungseinrichtung, bspw. der Spritze oder Pipette, aufgesogen wird. Das Herstellen der Reaktionsmischung erfolgt in diesem Fall innerhalb der Überführungseinrichtung. Ein Vermischen der Reaktionsflüssigkeit mit der zu analysierenden Probe kann bspw. dadurch erreicht werden, dass im Anschluss an die gewünschte Menge Probe ein Volumenanteil Luft in die Oberführungseinheit eingesaugt wird. Die Luft durchströmt im Aufsteigen die Reaktionsmischung und führt somit zu einer guten Durchmischung der Reaktionspartner.

Nachdem die Reaktionsmischung hergestellt ist, wird diese den ihr entsprechenden Inkubationsbedingungen ausgesetzt, um eine Reaktion der Reaktionspartner, insbesondere eine Bindung der Rezeptormoleküle an die entsprechenden Akzeptormoleküle, zu erreichen. Im Fall des Nachweises von Antibiotika in Milch durch bekannte Rezeptormoleküle erfolgt die Reaktion Insbesondere durch Einstellen einer vorgebbaren Temperatur für eine vorgebbare Zeit bzw. durch Ablauf eines vorgebbaren Zeit-Temperatur-Profils. Typische Reaktionsbedingungen für den Nachweis von Antibiotika in Milch durch Rezeptormoleküle sind bspw. 3 Minuten bei 47°C. Die Reaktionszeiten und Temperaturen bzw. Temperaturverläufe sind jedoch je nach Reaktion zu wählen und können bel anderen Nachweissystemen von dem genannten Profil abweichen. Die Herstellung der Reaktionsbedingungen erfolgt mittels einer Inkubationsvorrichtung, die bspw. eine steuerbare Temperiereinrichtung, insbesondere ein programmierbarer Thermoblock mit Zeitschaltuhr sein kann. Das Reaktionsgefäß ist hierzu in die Inkubationseinrichtung einzubringen oder befindet sich alternativ von Anfang an in der Inkubationseinrichtung. Insbesondere kann die Überführungseinrichtung, innerhalb derer sich eine Reaktionsmischung befindet, zumindest mit dem die Reaktionsmischung enthaltenden Teil innerhalb der Inkubationseinrichtung angeordnet sein, so dass die Reaktion innerhalb der Überführungseinrichtung, insbesondere der Spritze oder Pipette, stattfinden kann.

Eine Detektion der Reaktion kann direkt oder indirekt erfolgen. Mit direkter Detektion ist im vorliegenden Zusammenhang eine Untersuchung der Reaktionsmischung ohne Durchführung eines weiteren Verfahrensschrittes gemeint. Bspw. können sich während einer Reaktion die optischen Eigenschaften (Farbe, Absorption, Fluoreszenz, otc.) der Reaktionsmischung in spezifischer Weise verändern. Derartige Veränderungen können direkt mithilfe entsprechender Detektionseinrichtungen, bspw. Absorptions-, Fluoreszenzspektrometer oder Farbsensor detektiert werden. Hierzu kann bspw. das Reaktionsgefäß oder die die Reaktionsmischung enthaltende Überführugseinrichtung in die Detektionseinrichtung eingebracht werden. Alternativ kann die Reaktionsmischung mittels einer Überführungseinrichtung dem Reaktionsgefäß entnommen werden und/oder aus der Überführungseinrichtung in ein Detektionsgefäß (bspw, eine Küvette) überführt werden.

Für eine indirekte Detektion wird die Reaktionsmischung nach erfolgter Reaktion einer weiteren, detektierbaren Reaktion unterzogen, bspw. der Reaktion mit einem Indikator. Dies ist dann nötig, wenn die eigentliche Reaktion (bspw. die Bindung der Rezeptormoleküle an die Antibiotikamoleküle) keine leicht detektierbaren Veränderungen in der Reaktionsmischung zur Folge hat. Eine indikatorreaktion dient im Allgemeinen auch bei einer direkt detektierbaren Reaktion der Vereinfachung bzw. Verstärkung der Detektion. Bspw. kann ein Farbindikator verwendet werden, der bei Vorhandensein einer bestimmten, durch die Reaktion erzeugten Umgebung (bspw. Überschreiten eines bestimmten pH-Wertes, etc.) seine Farbe ändert. Der (Farb-)indikator oder mehrere (Farb-)Indikatoren können in Lösung oder immobilisiert, bspw. auf Trägern (indikatorstreifen) angewendet werden. Durch Immobilisierung und insbesondere durch das Zusammenwirken unterschiedlicher Indikatoren und durch Musterbildung können komplexe Nachweise und auch parallele Nachweise mehrerer Reaktionen erfolgen.

Die indikatorlösung oder -streifen können nach erfolgter Hauptreaktion in die Reaktionsmischung eingebracht werden oder in einem Indikatorgefäß bereitgestellt sein, in das die Reaktionsmischung nach Ende der Reaktion überführt wird. In einer bevorzugten Ausführungsform der Erfindung ist die Indikatoreinrichtung ein Indikatorstreifen, der mit einem klaren Kunststofffolienformteil abgedeckt ist, wobei das Kunststofffolienformteil eine Höhlung zur Aufnahme der Reaktionsmischung zur Verfügung stellt (Sichthülle bzw. Blister). Die Reaktionsmischung kann bspw. in diese Hülle eingebracht werden, indem die Rückwand (der Indikatorstreifen) mit der Kanüle durchslochen wird, um mit Hilfe der Überführungseinrichtung die Reaktionsmischung in die Höhlung des Blisters einzubringen.

Gemäß einer alternativen bevorzugten Ausführungsform der Erfindung ist die Einrichtung mit einer Reaktionskomponente eine auf einem Träger immobilisierte Reaktionskomponente (Teststreifen). Die Reaktionskomponente selbst ist in der vorliegenden Beschreibung bereits ausführllch beschrieben. Eine Immobilisierung von Reaktionskomponenten wie bspw. Bindungsmolekülen auf einem Träger ist im Stand der Technik bekannt.

Das erfindungsgemäße Zusammenführen zumindest eines Teilvolumens der zu analysierenden Probe mit der Reaktionskomponente kann im Fall einer immobilisiert auf einem Träger vorliegenden Reaktionskomponente aus besonders einfache Weise erfolgen. Der Teststreifen kann bspw. ganz oder zu einem großen Teil in die zu analysierende Probe eingetaucht werden. Alternativ kann er eine saugfähige Oberfläche aufweisen oder derart mit einer Folie abgedeckt sein, dass ein Eindringen der zu analysierenden Probe mittels Kapillarwirkung erfolgen kann. Der Teststreifen kann hierzu bspw. nur zu einem kleinen Teil in die zu analysierende Probe eingetaucht werden. Auch kann dem Teststreifen mittels einer Überführungseinrichtung ein Probevolumen der zu analysierenden Probe zugeführt werden. In diesem Fall kann der Teststreifen bspw. ähnlich blisterartig aufgebaut sein wie ein bereits beschriebener Indikatorstreifen. Ein Teststreifen kann, nachdem er mit der zu analysierenden Probe in Kontakt gebracht ist, den der entsprechenden Nachweisreaktion entsprechenden Bedingungen ausgesetzt sein, bspw. indem er einem bestimmten Zeit-Temperatur-Profil ausgesetzt wird.

Besonders bevorzugt ist ein beschriebener Teststreifen gleichzeitig der Indikatorstreifen. Bspw. können auf einem Träger gleichzeitig die Reaktionskomponente und der Indikator immobilisiert sein, so dass der gesamte Nachweisvorgang an einem einzigen Test- bzw. Nachweisstreifen erfolgen kann.

In dieser Ausführungsvariante der Erfindung entfällt zumindest ein Überführen von Reaktionsflüssigkeit und ein Mischvorgang, was das Verfahren deutlich vereinfacht.

in den beschriebenen Ausführungsformen der Erfindung kann die Detektion der Indikatorreaktion mit einer entsprechenden Detektionseinrichtung erfolgen. Insbesondere kann die Detektionseinrichtung eine Einrichtung zur optischen Erfassung, bspw. eine Kamera oder auch ein Spektrometer sein. Bevorzugt ist sie ein Scanner, der mit Hilfe von Sensoren, bspw. Farbsensoren einen Indikator- bzw. Teststreifen abtastet und anschließend die so qewonnenen Daten als elektrischen Messwert liefert bzw. in digitaler Form an eine Speichereinrichtung weitergibt.

Die Speichereinrichtung kann bspw. eine Codemarke oder ein RFID-Etikett (TAG) zur Radio Frequency Identification (Funkerkennung) sein, welches am Behälter angeordnet und diesem direkt zugeordnet ist. Der Behälter ist hierdurch optisch/elektronisch identifizierbar und die Analysedaten sind ihm direkt zugeordnet. Die Speicherung kann alternativ oder zusätzlich in eine Datenbank erfolgen. Diese kann auf einem Speichermedium abgelegt sein, welches sich ebenfalls an Bord des Sammcltransportfahrzeuges befindet, und/oder in einer entfernten Datenspeichereinrichtung abgelegt sein, an die die neu ermittelten Datensätze übermittelt werden, bspw. per Funk. Auf diese Weise kann die Sammelstelle bereits vorab über die schlechte Qualität einer Lieferung informiert sein und entsprechende Maßnahmen ergreifen. Ins - besondere ist somit die Zusammenführung aller Daten aller Sammeltransportfahrzeuge auf einfache Weise möglich und erfordert keine tehleranfällige manuelle Übertragung.

Die bevorzugte Kombination einer teststreifenförmigen bzw. blisterförmigen Farbindikatoreinrichtung mit einem Farbscanner bietet praktische Vorteile. Teststreifen und Blister können auf einfache Weise gefertigt und gelagert werden. Die Detektion der Farbreaktion mittels Farbscanner auf dem Indikator- bzw. Teststreifen kann direkt oder durch eine durchsichtige Abdeckung hindurch erfolgen. Die Teststreifen bzw. Blister können auf einfache Weise an der Vorrichtung angebracht sein, bspw. an einen nach außen weisenden Seitenwandbereich eines Probenbehälters angeklebt sein. Auf diese Weise ist auch die Zuordnung des Analysenergebnisses zur richtigen Probe auf einfache Weise gewährleistet. Nach Beendigung der Nachweise können die Teststreifen oder Blister entsorgt werden.

Gemäß einer bevorzugten Ausführungsform der Erfindung ist das beschriebene Verfahren automatisiert. Hierzu sind die beteiligten Behälter in einer beweglichen Halterung bereitgestellt, die eine Positionierung der Behälter ermöglicht. Die Positionierung ist bspw, zum Befüllen der Behälter bzw, zur Entnahme von Behälterinhalten mittels Überführungseinrichtungen oder zum Scannen der Indikatoreinrichtung notwendig. Die Halterung kann bspw. ein drehbares und/oder schwenkbares Halterungskarussell oder ein linear verschiebbarer Träger mit linearer Behälteranordnung sein.

Zudem sind sowohl die Einrichtung zur Entnahme der Analysenprobe als auch die Überführungseinrichtung zur Entnahme der vorgebbaren Mengen an Reaktionsflüssigkeit und Analysenprobe schwenkbar sowie heb- und senkbar. Diese Bewegungen sind über Dreh-Linearantriebe vermittelbar und steuerbar.

Des Weiteren ist die Betätigung der Überführungseinrichtung zur Entnahme der vorgebbaren Mengen an Reaktionsflüssigkeit und Analysenprobe automatisiert. Bspw. kann der Kolben der Einweg- oder Mehrwegspritze mittels einer mit dem Kolben verbundenen Linearantriebseinheit bewegbar sein. Die Vorgabe der entsprechend aufzunehmenden bzw. abzugebenden Volumina erfolgt über die Vorgabe von entsprechenden Hub- und Senkwcgcn.

Die Inkubationseinrichtung, bspw, ein Thermoblock, kann erfindungsgemäß gleichzeitig als Halterung für die Überführungseinrichtung dienen bzw. eine Einrichtung zur Halterung und/oder Betätigung der Überführungseinrichtung aufweisen. Hierbei kann die Überführungseinrichtung. bspw. die Spritze, zumindest teilweise innerhalb des Thermoblocks angeordnet sein. Überführungs- und inkubationseinrichtung sind in diesem Fall gemeinsam schwenkbar und heb- bzw. senkbar.

Die beschriebene Analyse kann mehrmals hintereinander durchgeführt werden. Dabei kann einerseits aus ein und demselben Liefergut mehrmals eine Analysenprobe entnommen und jeweils getrennt voneinander analysiert werden, um eine höhere Ergebnissicherheit zu gewährleisten. Außerdem können mehrere Lieferungen hintereinander gesammelt und geprüft werden. In beiden Fällen ist es erforderlich, mehrere saubere, insbesondere sterile, leere Behälter bereitzustellen. Auch können mehrere, jeweils einem leeren Behälter zugeordnete Behälter mit Reaktionsflüssigkeit bereitgestellt sein. Dies hat insbesondere bei einem automatisierten Verfahren den Vorteil, dass für jede Einzelanalyse die identischen Positionierungsbewegungen durchgeführt werden können. Bei Bereitstellung nur eines einzigen Behälters mit Reaktionsflüssigkeit ist die Bewegung, die die Halterung zur Entnahme aus diesem Behälter durchführen muss, in jedem Analysezyklus eine andere. Ein derartiges Vorgehen ist zwar komplizierter, aber ebenso möglich.

Ähnliches gilt für das Bereitstellen von Reinigungsflüssigkeit, die nach Beendigung einer jeden Einzelanalyse benötigt wird, um die Apparatur. insbesondere die Überführungseinrichtung und insbesondere die Überführungseinrichtung die als Reaktionsgefäß dient, zu reinigen. Das Unterlassen eines derartigen Reinigungsschrittes kann zu verfälschten Ergebnissen und zur Kontamination von Liefergutproben führen.

Da das erfindungsgemäße Verfahren im Sammeltransportfahrzeug durchgeführt werden kann, kann das Analyseergebnis unmittelbar dem Führer des Sammeltransportfahrzeuges zur Verfügung stehen. Dieser ist somit in die Lage versetzt, entsprechende Maßnahmen ergreifen zu können, falls das Ergebnis nicht zufrieden stellend ausfällt. Bspw. kann bei Nachweis von unzulässigen Mengen an Antibiotika in der Milch sofort die Aufnahme des Lieferguts in den Sammeltank gestoppt und somit die Qualität der Gesamtmenge gesichert werden. Dies kommt zum einen der Qualität des Endproduktes zugute, zum anderen werden wirtschaftliche Verluste vermleden. Durch das automatisierte und zeitlich bereits vor oder während des Überführens des Lieferguts von dem Lieferbehälter in den Sammeltransportbehälter durchführbare Verfahren verliert der Fahrer zudem keine Zeit; die Qualität des aufgenommenen Liefergutes wird automatisch und ohne dass der Fahrer explizit daran denken muss, geprüft, und wenn - wie es in der Regel der Fall ist - die Qualität des aufgenommenen Gutes in Ordnung ist, kann der Fahrer ohne zeitliche Verzögerung zur nächsten Lieferstelle weiterfahren. Zudem wird durch die Automatisierung eine fehlerhafte Durchführung der Analyse ausgeschlossen.

Eine bevorzugte Ausführungsform der Erfindung dient der weiteren Sicherung der Analysenergebnisse. Hierzu weist die Halterung für die Behälter eine Abdeckung auf, die die Behälter von oben in ihren Positionen fixiert. Insbesondere kann die Abdeckung an der Halterung befestigt sein, so dass ein Abnehmen der Abdeckung und somit ein Herausnehmen der Behälter nicht auf einfache Weise möglich ist. Auch ein Versiegeln bzw. Verplomben des Halterungsverschlusses ist möglich. Auf diese Weise kann ein menschliches Einwirken auf das Analysenergebnis nahezu vollständig ausgeschlossen werden.

Alle genannten Einrichtungen sind vorzugsweise an einer gemeinsamen Bodenplatte befestigt, so dass sie eine bauliche Einheit bilden.

Die Steuerung aller Einrichtungen erfolgt bevorzugt über eine gemeinsame programmierte bzw. programmierbare Steuereinrichtung, um koordinierte Abläufe sicherzustellen.

Zur sicheren Unterbringung der Vorrichtung kann diese in einem speziellen Gehäuse untergebracht sein, das bspw. durch einen Klappdeckel zugänglich ist.

### Ausführungsbeispiel

Die Erfindung wird nachfolgend anhand eines bevorzugten Ausführungsbeispiels unter Bezugnahme auf die beiliegenden Zeichnungen näher erläutert. Das Beispiel dient zur Illustrierung der Erfindung, ist jedoch in keiner Weise einschränkend zu verstehen. Gleiche Teile sind grundsätzlich mit gleichen Bezugsziffern versehen und werden teilweise nicht mehrfach erläutert.
Figur 1 verdeutlicht in schematischer Darstellung das erfindungsgemäße Verfahren.
Figur 2 zeigt eine Perspektivansicht der Vorrichtung zur Durchführung des erfindungsgemäßen Verfahrens.
Figur 3 verdeutlicht den Verschlussmechanismus der Halterung der Vorrichtung zur Durchführung des erfindungsgemäßen Verfahrens.

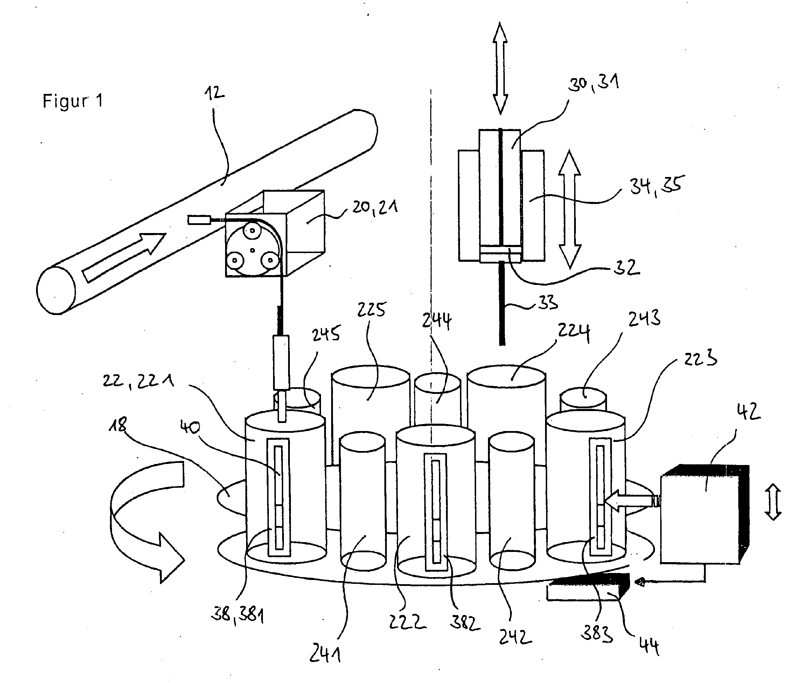

Figur 1 verdeutlicht in schematischer Darstellung das erfindungsgemäße Verfahren. Ein fließfähiges Liefergut wird durch eine Überführungsleitung 12 von einem Anlieferungsbehälter in einen Sammelbehälter überführt. Eine Entnahmeeinrichtung 20, im dargestellten Fall eine Peristaltikpumpe 21, ist so an diese Überführungsleitung 12 angeschlossen, dass darüber eine vorgebbare Menge an Liefergut, bspw. Rohmilch, abgezweigt werden kann. Die Entnahmevorrichtung 20 befördert dieses abgezweigte repräsentative Probenvolumen in einen zuvor bereitgestellten, sauberen, insbesondere sterilen, leeren Behälter 22. Im dargestellten Beispiel sind fünf derartige Behälter 221, 222, 223, 224 und 225 bereitgestellt, denen jeweils ein weiterer Behälter 241, 242. 243. 244 und 245 zugeordnet ist, in dem eine Reaktionsflüssigkeit bereitgestellt ist. Eine von der Entnahmeeinrichtung 20 unabhängige Überführungseinrichtung 30 erlaubt die Enthahme vorgebbarer Mengen aus den bereitgestellten Behältern 22, 24. Im dargestellten Beispiel ist die Überführungseinrichtung 30 eine Spritze 31. Diese ist mit ihrem unteren Bereich in eine Inkubationseinrichtung 34, im speziellen Fall in einen Thermoblock 35, eingepasst. Durch Heben und Senken des Spritzenkolbens 32 können vorgebbare Mengen der unterschiedlichen Behälterinhalte in den zylinderförmigen Hohlraum der Spritze eingesaugt bzw. wieder herausgedrückt werden. Gemäß dem erfindungsgemäßen Verfahren werden für eine Analyse einer Probe zunächst 25 µl der Reaktionsflüssigkeit aus einem Behälter 241 in die Spritze 31 aufgenommen. Im Falle des Nachweises von Antibiotika in Rohmilch ist es bspw. üblich, eine Reaktionsflüssigkeit zu verwenden, die Rezeptormoleküle aufweist, die an Antibiotikamoleküle binden können. Anschließend werden zusätzlich 150 µl der dem Behälter der Reaktionsflüssigkeit 241 zugeordnelen zu analysierenden Probe aus dem Behälter 221 aufgenommen. Die beiden Flüssigkeiten werden im Hohlraum der Spritze 31 miteinander vermischt, indem der Spritzenkolben 32 nochmals gehoben wird, während die Spritzenöffnung bzw. Kanüle 33 frei liegt. Auf diese Weise wird Luft in die Spritze 31 gesaugt, die innerhalb des Spritzenhohlraums nach oben steigt und dabei eine Verwirbelung und Vermischung der Reaktionspartner bewirkt. Gleichzeitig oder unmittelbar anschließend sorgt die Inkubationseinrichtung 34 für die Einhaltung einer definierten Temperatur über eine definierte Zeitspanne hinweg, um die in der Spritze 31 befindlichen Reaktionspartner zur Reaktion zu bringen Bspw. wird die Reaktionsmischung in der Spritze zur Bindung von Rezeptormolekülen an Antibiotikemoleküle in Milch für 3 Minuten auf 47°C erwärmt. Im Anschluss an den Inkubationsschritt wird die Reaktionsmischung aus der Überführungseinrichtung 30 direkt in eine ihr zugeordnete, ungebrauchte indikatoreinrichtung 381 eingebracht. Die Indikatoreinrichtung 381 ist an der Seitenwand des Behälters 221 angebracht und weist einen Indikatorstreifen 40 auf, der auf eine erfolgte Reaktion in der Reaktionsmischung reagiert. Bspw. kann der Indikatorstrelfen 40 Immobilisierte Indikatormoleküle aufweisen, die in der Lage sind, den Rezeptor-Antikörperkomplex zu binden, nicht jedoch den Rezeptor alleine. Die Bindung des Komplexes kann das Lichtabsorptionsspektrum des Indikatormoleküls derart verändern, dass es zu einer sichtbaren Farbreaktion kommt. Die Indikatoreinrichtung 381 ist derart von außen einsehbar, dass diese Indikatorreaktion von außen detektierbar ist. Hierzu weist die erfindungsgemäße Vorrichtung eine Detektionseinrichtung 42 auf, die bspw. ein Farbscanner ist und den Indikatorstreifen 40 abtastet. Die jeweilige Indikatoreinrichtung 38 wird zu diesem Zweck vor der Detektionseinrichtung 42 positioniert. Die jeweilige Positionierung der Behälter 22, 24 bzw. der Indikatoreinrichtung 38 erfolgt durch Drehung der Halterung 18. Die Detektionseinrichtung 42 leitet das Analysenergebnis an eine Datenspeichereinrichtung 44 weiter.

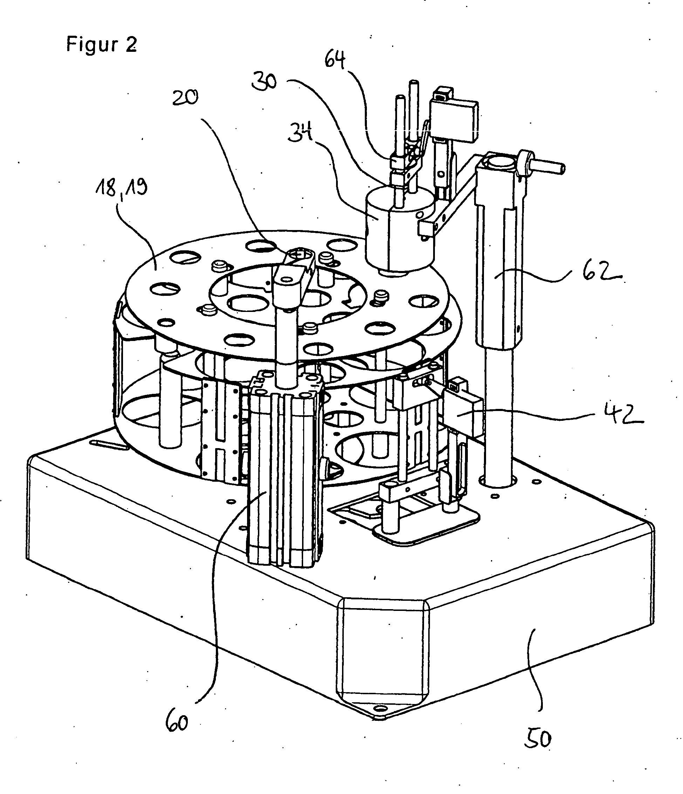

Figur 2 verdeutlicht eine konkrete Ausführungsform einer Vorrichtung, mit der die Durchführung des beschriebenen Verfahrens ermöglicht ist. Zusätzlich zu den bereits in Zusammenhang mit Figur 1 gegebenen Erläuterungen sind hier insbesondere die Vorkehrungen zur Automatisierung verschiedener Verfahrens schritte sichtbar.

Die Halterung 18 für die Behälter 22, 24 (nicht dargestellt) ist ein drehbares Halterungskarussell 19, das über einen Motor bewegt wird. Durch die Drehung des Karussells 19 können die Behälter 22 jeweils in eine für die Entnahmeeinrichtung 20 zugängliche Position gebracht werden, um die Probennahme zu ermöglichen. Des Weiteren können die Behälter mit der zu analysierenden Probe 22 und die Behälter für die Reaktionsflüssigkeiten 24 jeweils in eine für die Überführungseinrichtung 30 zugängliche Position gebracht werden, um jeweils vorgebbare Mengen der Behälterinhalte zu entnehmen. Schließlich können die Indikatoreinrichtungen 38 durch Drehung des Karussells 19 jeweils für ihre Befüllung in eine für die Überführungseinrichtung 30 zugängliche Position gebracht werden sowie jeweils vor der Detektionseinrichtung 42 positioniert werden.

Die Entnahmeeinrichtung 20 sowie die Überführungseinrichtung 30 weisen an ihren den Behältern zugewandten Öffnungen jeweils eine Kanüle 17, 33 auf, mittels derer Bereiche in den Abdeckungen 23, 25 der Behälter 22, 24 durchstochen werden können, um Behälterinhalte zu entnehmen oder diese einzufügen. Die durchstechbaren Bereiche in den Abdeckungen 23, 25 bestehen bspw. aus Silikon, da sich diese nach Entfernen der Kanüle 17, 33 selbstständig wieder luftdicht verschließen.

Die Entnahmeeinrichtung 20 sowie die Überführungseinrichtung 30 sind mittels Schwenk- und Linearantrieben 60, 62 schwenkbar sowie heb- und senkbar. Durch die Schwenkbewegungen können die Einrichtungen 20, 30 derart zu den Behältern 22, 24 bzw. den Behälterabdeckungen 23, 25 positioniert werden, dass ein anschließendes Senken der jeweiligen Einrichtung 20, 30 ein Durchstechen der Abdeckung 23, 25 mit der Kanüle 17, 33 zur Folge hat. Durch Heben der Entnahmeeinrichtung 20 bzw. der Überführungseinrichtung 30 wird die jeweilige Kanüle 17, 33 wieder aus dem jeweiligen Behälter 22, 24 entfernt.

Mittels der Entnahmeeinrichtung 20 kann bei eingestochener Kanüle 17 das Probenvolumen in den Behälter 22 gepumpt werden. Mittels der Überführungseinrichtung 30 kann bei genügend hohem Flüssigkeitsstand in dem jeweiligen Behälter 22 bzw. 24 bzw, bei genügend langer Kanüle 33 in dieser Position Behälterinhalt entnommen werden. Die geschieht durch Heben des Spritzenkolbens 32. Durch Senken des Spritzenkolbens 32 kann der Spritzeninhalt wieder abgegeben werden. Die Bewegung des Spritzenkolbens 32 erfolgt über eine Betätigungseinrichtung 64, die mit einem Linearantrieb ausgestattet ist. Die Halterung der Spritze 31 ist im gezeigten Beispiel in die Inkubationseinrichtung 34 integriert. Die Halterung und Betätigungseinrichtung 64 des Spritzenkolbens 32 ist oberhalb der Inkubationseinrichtung 34 angeordnet.

Gemäß dem erfindungsgemäßen Verfahren werden also zunächst der Behälter 241 durch Drehung des Halterungskarussells 19 sowie durch Schwenken der Überführungseinrichtung 30 zueinander in Position gebracht. Durch Senken der Überführungseinrichtung 30 durchsticht die Kanüle 33 die Abdeckung 251. Durch Heben des Spritzenkolbens 32 mittels der Betätigungseinrichtung 64 wird dem Behälter 241 die gewünschte Menge Reaktionsflüssigkeit entnommen. Anschließend wird die Überführungseinrichtung 30 angehoben, um die Kanüle 33 aus dem Behälter 241 herauszuziehen. Im Anschluss daran wird der Behälter 221 durch Drehung des Halterungskarussells 19 so positioniert, dass beim Absenken der Überführungseinrichtung 30 die Kanüle 33 die Abdeckung 231 durchsticht. Durch Heben des Spritzenkolbens 32 mittels der Betätigungseinrichtung 64 wird dem Behälter 221 die gewünschte Menge zu analysierender Flüssigkeit entnommen. Nach dem Entfernen der Kanüle 33 aus dem Behälter 221 durch I leben der Überführungseinrichtung 30 wird nochmals mittels Betätigungseinrichtung 64 der Spritzenkolben 32 angehoben. Auf diese Weise wird Luft in die Spritze 31 gesaugt und der Spritzeninhalt vermischt. Die in der Spritze befindliche Reaktionsmischung kann nun mittels Inkubationseinrichtung 34 erwärmt und anschließend die Reaktion überprüft werden.

Im Beispiel erfolgt die Detektion der Reaktion indirekt über einen Indikator. Das Befüllen der Indikatoreinrichtung 38 erfolgt auf die bereits beschriebene Weise. Allerdings weist die Indikatoreinrichtung 38 im Beispiel keinen Silikonverschluss auf. Die Indikatoreinrichtung 38 umfasst einen relativ festen Träger, auf dem ein Indikator immobilisiert ist. Dieser Träger ist mit einem durchsichtig klaren Kunststofffolienformteil bedeckt, so dass zwischen dem Träger und dem Kunststofffolienformteil ein Hohlraum zur Aufnahme der Reaktionsmischung entsteht. Die Reaktionsmischung wird eingefüllt, indem die Indikatoreinrichtung 38 bzw. der Behälter 22, an dessen Außenwand sie sich befindet, über die Drehung des Karussells 19, und die Überführungseinrichtung 30 durch Schwenken und eine lineare Bewegung senkrecht zur Einstechbewegung zueinander positioniert werden, so dass die Indikatoreinrichtung bei einem Absenken der Überführungseinrichtung 30 vorzugsweise an der Trägerwand durchstochen wird. Durch Senken des Spritzenkolbens 33 kann daraufhin die Reaktionsmischung in die Indikatoreinrichtung 38 eingefüllt werden.

Um koordinierte Ablaufe sicherzustellen erfolgt die Steuerung der beweglichen Einrichtungen (der Halterung 18, 19, der Entnahmeeinrichtung 20 und der Überführungseinrichtung 30 einschließlich der Inkubationseinrichtung 34, 35) über eine gemeinsame programmierte bzw. programmierbare Steuereinrichtung 54, die insbesondere die genannten Dreh-Lineareinheiten 60, 62 bzw. die Lineareinheit, welche die Betätigungseinrichtung 64 für den Kolben 32 anspricht, steuert.

Alle Einrichtungen der Vorrichtung sind auf einer gemeinsamen Bodenplatte 50 angeordnet und bilden so eine bauliche Einheit.

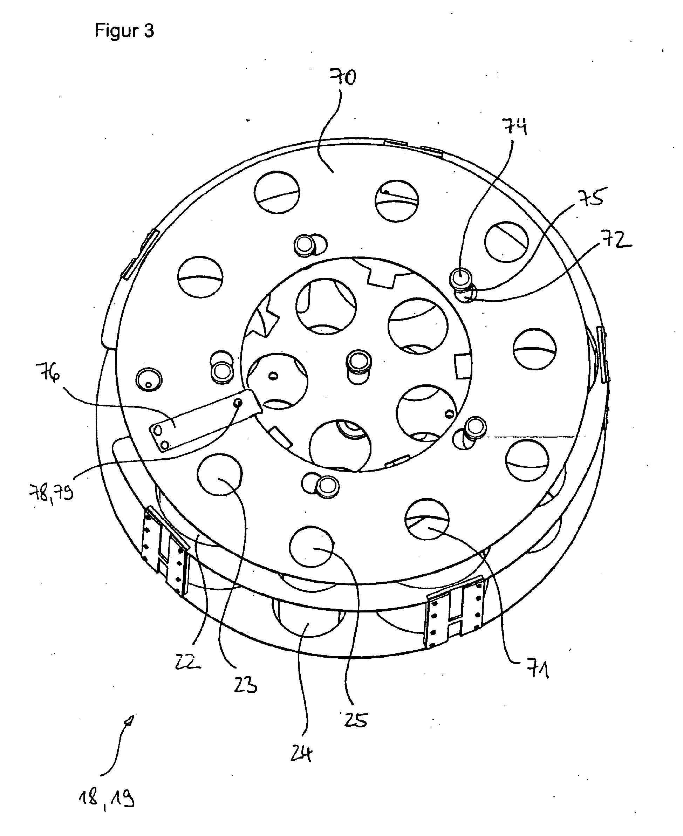

Figur 3 verdeutlicht, wie die Halterung 18 der Vorrichtung abgedeckt sein kann, um die Behälter in ihren jeweiligen Positionen zu sichern und ggf. eine Manipulation der Messdaten durch Austauschen von Behältern zu unterbinden. Ein Halterungskarussell 19 kann eine ringförmige Abdeckung 70 aufweisen, die bspw. langlochartige Aussparungen 72 aufweist, deren Breite nach einer Seite hin abnimmt. Am Halterungskarussell 19 können den Aussparungen 72 zugeordnete Zapfen 74 angeordnet sein, die jeweils eine umlaufende Nut 75 aufweisen. Zur Befestigung der Abdeckung 70 auf dem Halterungskarussell 19 können somit die Aussparungen 72 auf die Zapfen 74 aufgesteckt werden. Durch eine anschließende leichte Drehung der Abdeckung 70 greift jeweils die Nut 75 eines Zapfens 74 in die Randabschnitte des schmaleren Endes der Aussparung 72 ein und verhindert somit ein Abheben der Abdeckung 70. Bspw. eine Blattfeder 76 o, ä. kann die Abdeckung 70 in dieser Lage fixieren, indem sie den breiteren Teil eines Langloches 72 abdeckt und somit eine Drehung der Abdeckung 70 unterbindet.

Die ringförmige Abdeckung 70 weist einen derartigen Durchmesser und eine derartige Breite auf, dass sie im auf dem Halterungskarussell 19 befestigten Zustand über den Behältern 22, 24 liegt. Um weiterhin eine Zugänglichkeit der Behälter 22, 24 für die Entnahme- bzw. Überführungseinrichtungen 20, 30 zu gewährleisten, weist die Abdeckung 70 an entsprechenden Stellen genügend große Aussparungen 71 auf, durch die hindurch die Behälterabdeckungen 23, 25 zugänglich sind.

Die Blattfeder 76 sowie die Abdeckung 70 können jeweils Bohrungen 78, 79 aufweisen, die in der fixierten Lage der Abdeckung 70 auf dem Karussell 19 fluchtend übereinander liegen. Durch diese Bohrungen 78. 79 hindurch kann ein Befestigungsmittel, bspw. ein Draht, gezogen sein, dessen aus den Bohrungen herausragenden Abschnitte zudem mittels einer Plombe verbunden sein können. Auf diese Weise wird ein ungerechtfertigtes Entfernen der Abdeckung 70 verhindert bzw. kann erkannt werden,

### Bezugszeichenliste

- 12: Überführungsleitung
- 14: Anlieferungsbehälter
- 16: Sammelbehälter
- 17: Kanüle
- 18: Halterung
- 19: Halterungskarussell
- 20: Entnahmeeinrichtung
- 21: Peristaltikpumpe
- 22: Behälter zur Aufnahme der zu analysierenden Probe
221 erster Behälter zur Aufnahme der zu analysierenden Probe
222 zweiter Behälter zur Aufnahme der zu analysierenden Probe etc.
23 Abdeckung für den Behälter zur Aufnahme der zu analysierenden Probe
231 Abdeckung für den ersten Beträlter zur Aufnahme der zu analysierenden Probe
232 Abdeckung für den zweiten Behälter zur Aufnahme der zu analysierenden Probe etc,
- 24: Behälter zur Bereitstellung der Reaktionsflüssigkelt
241 erster Behälter zur Bereitstellung der Reaktionsflüssigkeit
242 zweiter Behälter zur Bereitstellung der Reaktionsflüssigkeit etc.
- 25: Abdeckung für den Behälter zur Bereitstellung der Reaktionsflüssigkeit
251 Abdeckung für den ersten Behälter zur Bereitstellung der Reaktionsflüssigkeit
252 Abdeckung für den zweiten Behälter zur Bereitstellung der Reaktionsflüssigkeit etc.
- 30: Überführungseinrichtung
- 31: Spritze
- 32: Spritzenkolben
- 33: Kanüle
- 34: Inkubationseinrichtung
- 35: Thermoblock
- 38: Indikatoreinrichtung
381 die dem ersten Behälter zugeordnete Indikatoreinrichtung
382 die dem zweiten Behälter zugeordnete Indikatoreinrichtung etc.
- 40: Indikatorstreifen
- 42: Detektionseinrichtung
- 44: Datenspeichereinrichtung
- 50: Bodenplatte
- 54: Steuereinrichtung
- 60: Dreh-Linear-Antrieb für die Entnahmeeinrichtung
- 62: Dreh-Linear-Antrieb für die Überführungseinrichtung
- 64: Betätigungseinrichtung für den Spritzenkolben
- 70: ringförmige Abdeckung
- 71: Aussparung für Behälter
- 72: langlochförmige Aussparung
- 74: Zapfen
- 75: umlaufende Nut
- 76: Blattfeder
- 78: Bohrung in der Blattfeder
- 79: Bohrung in der Abdeckung

## Patentansprüche

1. Verfahren zur Überführung und gleichzeitiger Analyse fließfähiger Sammelgutmengen, insbesondere von Milchlieferungen, an Lieferstellen in Sammeltransportfahrzeugen, umfassend folgende Schritte:
• überführung des Liefergutes;
• Entnahme einer Probe des Lieferguts während des Überführens des Lieferguts von einem Lieferbehälter in einen Sammelgutbehälter;
**gekennzeichnet durch**
• das unmittelbare Durchführen mindestens einer Analyse der Probe zur Qualitätsbestimmung;
• Auswerten und Speichern des Reaktionsergebnisses mit Zuordnung zum Behälter (22); und
• Stoppen des überführens des Liefergutes bei ungewünschtem Ergebnis oder
• Fortsetzen des überführens des Liefergutes bei ausreichender Qualität Liefergutes.

2. Verfahren nach Anspruch 1, bei dem eine Reaktionskomponente zur Durchführung der Analyse zur Qualitätsbestimmung eine auf einem Träger immobilisierte Reaktionskomponente ist,
und das Zusammenführen zumindest eines Teilvolumens der zu analysierenden Probe mit der Reaktionskomponente die folgenden Schritte umfasst:
• Transport eines Probenvolumens der zu analysierenden Probe zu der immobilisierten Reaktionskomponente mittels Kapillarwirkung;
• ggf. Inkubation entsprechend vorgebbarer Inkubationsbedingungen.

3. Verfahren nach Anspruch 2, bei dem der Träger eine immobilisierte Indikatorkomponente für eine durch die Detektionseinrichtung (42) erkennbare Indikatorreaktion aufweist.

4. Verfahren nach Anspruch 1, bei dem eine Reaktionskomponente für die Qualitätsanalyse in einer Einrichtung bereit gestellt wird und das Bereitstellen der Einrichtung mit der Reaktionskomponente die folgenden Schritte umfasst:
• Bereitstellen von einem Behälter (24) mit einer Reaktionsflüssigkeit;
• Bereitstellen einer vorgebbaren Menge Reaktionsflüssigkeit;
• und bei dem das Zusammenführen zumindest eines Teilvolumens der zu analysierenden Probe mit der Reaktionskomponente die folgenden Schritte umfasst:
• Herstellen einer Reaktionsmischung durch Hinzugeben einer vorgebbaren Menge der zu analysierenden Probe zu der vorgegebenen Menge Reaktionsflüssigkeit;
• Inkubation der Reaktionsmischung entsprechend vorgebbarer Inkubationsbedingungen.

5. Verfahren nach Anspruch 4, bei dem die Reaktionsmischung nach der Inkubation und vor der Detektion in eine Indikatoreinrichtung (38) umfassend eine auf einem Träger (40) immobilisierte Indikatorkomponente für eine durch die Detektionseinrichtung (42) erkennbare Indikatorreaktion überführt wird.

6. Verfahren nach einem der voranstehenden Ansprüche, bei dem eine Befüllung der Behälter (22, 24) und/oder Entnahme aus den Behältern (22, 24) jeweils durch Durchstechen von sich selbst luftdicht verschließenden Behälterabdeckungen (23, 25) mittels einer Kanüle (17, 33) erfolgt und bei dem ggf. die Herstellung und Inkubation der Reaktionsmischung in einer Überführungseinrichtung (30) erfolgt.

7. Verfahren nach einem der Ansprüche 2 bis 6, bei dem die Reaktionskomponente Rezeptormoleküle zur Bindung an eine in der Analysenprobe zu detektierende Substanz aufweist.

8. Verfahren nach einem der voranstehenden Ansprüche, bei dem die Speicherung des Analyseergebnisses auf Codemarken oder RFID-Etiketten erfolgt, welche jeweils dem die analysierte Probe enthaltenden Behälter (22) zugeordnet sind.

## Claims

1. Method for transporting and directly analysing collected flowable material, in particular milk deliveries, to delivery places in general transport containers, comprising the following steps:
• transporting the delivery property;
• sampling of the delivery property during the transport of the delivered property from a delivery container into a general transport container;
**characterized in**
• the direct implementation of at least one analysis of the sample for quality determination;
• evaluating and storing the reaction result with allocation to the container (22); and
• stopping the transport of the delivery property in the event of an undesirable result or
• continuing the transport of the delivery property in the event of sufficient quality of the delivery property.

2. Method according to claim 1, wherein a reaction component for the implementation of the analysis for quality determination is an reaction component immobilized on a carrier means, and the bringing together of at least a partial volume of the sample with the reaction component comprises the following steps:
• transport of a sample volume of the sample to the immobilized reaction component by means of capillary action;
• incubation according to predetermined incubation requirements, if necessary.

3. Method according to claim 2, wherein the means is provided with an immobilized indicator component for an indicator reaction identifiable by the detector (42).

4. Method according to claim 1, wherein a reaction component for the quality analysis is provided in a device and the provision of the device with the reaction component comprises the following steps:
• providing a container (24) with a reaction liquid;
• providing a predetermined amount of reaction liquid;
• and wherein the bringing together of at least a partial volume of the sample with the reaction component comprises the following steps:
• producing a reaction mixture by adding a predetermined amount of the sample to the predetermined amount of reaction liquid;
• incubation of the reaction mixture according to predetermined incubation requirements.

5. Method according to claim 4, wherein the reaction mixture is transferred after the incubation and before the detection into an indicator device (38) comprising an indicator component immobilized on a carrier means (40) for an indicator reaction identifiable by the detector (42).

6. Method according to one of the aforesaid claims, wherein a filling of the containers (22, 24) and/or withdrawal from the containers (22, 24) is in each case carried out by piercing of hermetically self-sealed container covers (23, 25) by means of a cannula (17, 33), and wherein the production and incubation of the reaction mixture is carried out in a transportation device (30), if necessary.

7. Method according to one of the claims 2 to 6, wherein the reaction component contains receptor molecules for linkage to a substance to be detected in the analysis of the sample.

8. Method according to one of the aforesaid claims, wherein the storage of the analysis result is carried out on code labels or RFID-tags, which are in each case allocated to the container (22) containing the analysed sample.

## Revendications

1. Procédé pour le transfert et l'analyse simultanée de quantités de marchandises de groupage fluides, en particulier de livraisons de lait, à des points de livraison dans des véhicules de transport groupé, comprenant les étapes suivantes :
• transfert de la marchandise livrée ;
• prélèvement d'un échantillon de la marchandise livrée pendant le transfert de la marchandise livrée d'un récipient de livraison dans un récipient de marchandises de groupage ;
**caractérisé par**
• l'exécution directe d'au moins une analyse de l'échantillon en vue d'en déterminer la qualité ;
• exploitation et enregistrement du résultat de réaction avec affectation au récipient (22) ; et
• arrêt du transfert de la marchandise livrée si le résultat obtenu est indésirable, ou
• poursuite du transfert de la marchandise livrée si la qualité de la marchandise livrée est suffisamment bonne.

2. Procédé selon la revendication 1, dans lequel une composante réactionnelle pour l'exécution de l'analyse en vue de la détermination de la qualité est une composante réactionnelle immobilisée sur un support,
et le regroupement d'au moins un volume partiel de l'échantillon à analyser avec la composante réactionnelle comprend les étapes suivantes :
• transport d'un volume d'échantillon de l'échantillon à analyser vers la composante réactionnelle immobilisée par capillarité ;
• si nécessaire, incubation conformément à des conditions d'incubation pouvant être définies au préalable.

3. Procédé selon la revendication 2, dans lequel le support présente une composante indicatrice immobilisée pour une réaction d'indicateur détectable par l'équipement de détection (42).

4. Procédé selon la revendication 1, dans lequel une composante réactionnelle pour l'analyse de qualité est fournie dans un équipement, et la mise à disposition de l'équipement avec la composante réactionnelle comprend les étapes suivantes :
• mise à disposition d'un récipient (24) avec un liquide de réaction ;
• mise à disposition d'une quantité de liquide de réaction pouvant être définie au préalable ;
• et dans lequel le regroupement d'au moins un volume partiel de l'échantillon à analyser avec la composante réactionnelle comprend les étapes suivantes :
• génération d'un mélange de réaction par addition d'une quantité, pouvant être définie au préalable, de l'échantillon à analyser à la quantité de liquide de réaction prédéfinie ;
• incubation du mélange de réaction conformément à des conditions d'incubation pouvant être définies au préalable.

5. Procédé selon la revendication 4, dans lequel le mélange de réaction est transféré après l'incubation et avant la détection dans un équipement indicateur (38) comprenant une composante indicatrice immobilisée sur un support (40) pour une réaction indicatrice pouvant être détectée par l'équipement de détection (42).

6. Procédé selon l'une quelconque des revendications précédentes, dans lequel un remplissage des récipients (22, 24) et/ou prélèvements des récipients (22, 24) a lieu chaque fois par perforation de couvercles de récipients (23, 25) à auto-fermeture étanche au moyen d'une canule (17, 33) et dans lequel, si nécessaire, la génération et l'incubation du mélange de réaction ont lieu dans un équipement de transfert (30).

7. Procédé selon l'une quelconque des revendications 2 à 6, dans lequel la composante réactionnelle présente des molécules réceptrices pour la liaison à une substance à détecter dans l'échantillon d'analyse.

8. Procédé selon l'une quelconque des revendications précédentes, dans lequel l'enregistrement du résultat d'analyse se fait sur des marques codées ou étiquettes RFID qui sont chacune affectées au récipient (22) contenant l'échantillon analysé.
